## Europäisches Patentamt

## European Patent Office

## Office européen des brevets

(19)

(11) Veröffentlichungsnummer: **0 028 781**
**B1**

(12) **EUROPÄISCHE PATENTSCHRIFT**

(45) Veröffentlichungstag der Patentschrift:
04.08.82

(21) Anmeldenummer: 80106709.1

(22) Anmeldetag: 31.10.80

(51) Int. Cl.³: **C 07 C 119/048,** C 08 G 18/76,
C 09 J 3/12, C 09 J 3/16

(54) Polyisocyanatgemische der Diphenylmethanreihe und ihre Verwendung als Zusatzmittel in Klebstoffen.

(30) Priorität: **12.11.79 DE 2945656**

(43) Veröffentlichungstag der Anmeldung:
**20.05.81 Patentblatt 81/20**

(45) Bekanntmachung des Hinweises auf die Patenterteilung:
**04.08.82 Patentblatt 82/31**

(84) Benannte Vertragsstaaten:
**AT DE FR GB IT SE**

(56) Entgegenhaltungen:
**FR-A-1 553 295**
**GB-A-1 094 321**
**US-A-2 683 730**

(73) Patentinhaber: **BAYER AG, Zentralbereich Patente, Marken und Lizenzen, D-5090 Leverkusen 1, Bayerwerk (DE)**

(72) Erfinder: **Meyer, Rolf-Volker, Dr., Büchheimerstrasse 23, D-4150 Krefeld (DE)**
Erfinder: **Hombach, Rudolf, Dr., Johann-Janssen-Strasse 24, D-5090 Leverkusen 1 (DE)**
Erfinder: **Dollhausen, Manfred, Dr., Herzogenfeld 21, D-5068 Odenthal (DE)**

# 0 028 781

### Polyisocyanatgemische der Diphenylmethanreihe und ihre Verwendung als Zusatzmittel in Klebstoffen

Gegenstand der vorliegenden Erfindung sind Polyisocyanatgemische der Diphenylmethanreihe, gekennzeichnet durch

a) eine mittlere NCO-Funktionalität von 3,5 bis 5 und
b) einen Gehalt an
Zweikernisocyanaten von 0 bis 15 Gew.-%,
Dreikernisocyanaten von 10 bis 45 Gew.-%,
Vierkernisocyanaten von 5 bis 30 Gew.-% und
höherkernigen Polyisocyanaten von 30 bis 80 Gew.-%,

sowie die Verwendung dieser Polyisocyanatgemische als Zusatzmittel in Klebstoffen auf Basis von Natur- oder Synthesekautschuk oder Hydroxylgruppen aufweisenden Polyurethanen.

Polyisocyanate werden auf dem Klebstoffgebiet in großem Umfang zusammen mit Lösungen von Polymeren sehr unterschiedlicher Art verwendet.

So setzt man u. a. Lösungen aus Natur- oder Synthese-Kautschuk (u. a. Nitrilkautschuk, Chloroprenkautschuk) Lösungen von 4,4',4''-Triphenylmethan-triisocyanat zu und erreicht so eine günstigere Haftung an vielen zu klebenden Werkstoffen und eine höhere Beständigkeit der Klebungen bei Wärmeeinwirkung. Die Verwendbarkeit dieses Triisocyanates ist auf vielen Gebieten, z. B. in der Leder- und Kunstleder-verarbeitenden Industrie dadurch erheblich eingeschränkt, daß sich Klebungen mit diesem Triisocyanat, vor allem unter Lichteinfluß, dunkelviolett verfärben.

Bei verfärbungsempfindlichen Klebungen werden daher zur Vermeidung dieses Nachteils den Lösungen aus Natur- und Synthesekautschuk in der Praxis auch Polyisocyanat-Vernetzerkomponenten zugesetzt, die durch Anilin-Formaldehyd-Kondensation und anschließender Phosgenierung hergestellt werden (s. z. B. W. Siefgen in Justus von Liebigs Annalen der Chemie, 562, Seiten 75 – 136).

Diese technisch besonders leicht zugänglichen Produkte enthalten neben wechselnden Mengen höherkerniger Homologer als Hauptbestandteil Diphenylmethandiisocyanate, im wesentlichen 4,4'-Diphenylmethandiisocyanat. Die durchschnittliche NCO-Funktionalität dieser Isocyanatgemische liegt bei ca. 2,3 bis 3,3. Polyisocyanatgemische der Diphenylmethanreihe einer innerhalb dieses Bereichs liegenden NCO-Funktionalität werden auch in der US-PS 2 683 730 bzw. der GB-PS 1 094 321 beschrieben. Das einzige in der US-PS 2 683 730 konkret beschriebene Polyisocyanat weist ein Äquivalentgewicht von 136,1 auf. Dem Beispiel 1 der GB-PS 1 094 321 ist zu entnehmen, daß dieses Polyisocyanat der genannten US-Patentschrift ein Molekulargewicht von 400 aufweist, woraus sich eine mittlere NCO-Funktionalität von ca. 2,9 errechnet. Beiden Vorveröffentlichungen ist somit keinerlei Hinweis auf die nachstehend näher beschriebenen erfindungsgemäßen Polyisocyanatgemische einer mittleren NCO-Funktionalität von mindestens 3,5 bzw. deren besonders gute Eignung als Vernetzer für Klebstoffe zu entnehmen. Die gleiche Feststellung rechtfertigt sich im Hinblick auf die FR-PS 1 553 295, wo u. a. die bereits oben diskutierten Polyisocyanate als Zusatzmittel für Klebstoffe auf Polyurethanbasis erwähnt werden, ohne jeden Hinweis auf die nachstehend näher beschriebenen erfindungsgemäßen Polyisocyanate.

In der Praxis zeigt es sich, daß mit den genannten Polyisocyanatgemischen der Diphenylmethanreihe einer durchschnittlichen NCO-Funktionalität von 2,3 – 3,3 nicht die Festigkeit und Beständigkeit der Klebungen erhalten werden kann, die mit 4,4',4''-Triphenylmethan-triisocyanat zu erreichen sind. Dieser Nachteil wird besonders deutlich bei ihrer Verwendung mit den in der Praxis, z. B. in der Schuhindustrie zum Kleben von Gummimaterialien, sehr häufig eingesetzten und daher besonders wichtigen Klebstoffen aus Polychloropren, sowie für die zum Kleben von Polyvinylchlorid-Kunstledern praktisch unentbehrlich gewordenen und in sehr großem Umfange verwendeten Lösungen von Hydroxylpolyurethanen.

Ein wesentlicher Nachteil bei der Verwendung dieser Polyisocyanat-Gemische ist der vielfach unzureichende Schälwiderstand von Klebungen unmittelbar nach ihrer Herstellung in der Wärme. Für Anwendungen, bei denen sofort nach dem Klebevorgang eine Wärmebelastung auftritt, wie z. B. in der Schuhindustrie, wo unmittelbar nach der Montage der Sohlen unter erheblicher Wärmeentwicklung der Sohlenrand gefräst wird, ist ein ausreichender Schälwiderstand der frischen Klebungen jedoch unbedingt erforderlich.

Auch Polyisocyanate, hergestellt durch Polymerisation von Toluylendiisocyanaten, die in der Regel eine NCO-Funktionalität von $\geq 4$ aufweisen, haben sich trotz ihrer hohen Funktionalität in der Praxis nicht bewährt, da sie die Haftung von Klebstoffen an wenig klebfreudigen Materialien (z. B. Gummisohlen) nicht verbessern.

Überraschenderweise wurde nun gefunden, daß sich erheblich günstigere Kleberesultate erzielen lassen, wenn man als Vernetzer in Klebstoffen auf Basis von Natur- oder Synthesekautschuk oder auf Basis von Hydroxylgruppen aufweisenden Polyurethanen Polyisocyanatgemische der Diphenylmethanreihe mit einer mittleren NCO-Funktionalität von 3,5 bis 5 verwendet.

Im Rahmen der vorliegenden Erfindung sind unter »Polyisocyanatgemische der Diphenylmethan-

2

Reihe« sowohl die an sich bekannten Phosgenierungsprodukte von Anilin/Formaldehyd-Kondensaten als auch die entsprechenden, Methyl-substituierte aromatische Ringe aufweisenden Phosgenierungsprodukte von Toluidin/Formaldehyd-Kondensaten zu verstehen. Vorzugsweise hat der Begriff »Polyisocyanatgemische der Diphenylmethanreihe« jedoch die erstgenannte Bedeutung.

Im Rahmen der vorliegenden Erfindung kommt es wesentlich darauf an, daß die Polyisocyanatgemische eine mittlere NCO-Funktionalität von mindestens 3,5, im allgemeinen eine mittlere NCO-Funktionalität von 3,5 bis 5, vorzugsweise von 3,7 bis 4,5, aufweisen. Es handelt sich im allgemeinen um Polyisocyanatgemische, die 0 bis 15, vorzugsweise 0 bis 8 Gew.-% an Zweikern-, 10 bis 45, vorzugsweise 20 bis 30 Gew.-% an Dreikern-, 5 bis 30, vorzugsweise 10 bis 25 Gew.-% an Vierkernisocyanaten und 30 bis 80, vorzugsweise 40 bis 70 Gew.-% an höherkernigen Polyisocyanaten der Diphenylmethanreihe enthalten, so daß die genannte Bedingung bezüglich der NCO-Funktionalität erfüllt ist, und wobei sich die genannten Prozentsätze zu 100 Gew.-% ergänzen. Die o. g. mittlere NCO-Funktionalität läßt sich aus dem osmometrisch bestimmten mittleren Molekulargewicht der Polyisocyanatgemische und ihrem NCO-Gehalt errechnen.

Zur Herstellung der erfindungsgemäßen Polyisocyanatgemische kann man von großtechnisch durch Kondensation von Anilin und/oder Toluidin und Formaldehyd erhältlichen Polyaminen ausgehen, vorzugsweise von solchen Produkten, die bereits herstellungsbedingt einen höheren Anteil an Drei- und Mehrkernpolyaminen enthalten.

Durch destillative Entfernung leichter flüchtiger Anteile erhält man die Polyamingemische, die durch Phosgenierung nach bekannten Verfahren die erfindungsgemäß einzusetzenden Polyisocyanatgemische liefern.

Vorzugsweise phosgeniert man jedoch zunächst das technisch anfallende Polyamin-Gemisch und gewinnt die erfindungsgemäßen Polyisocyanat-Gemische durch anschließende Anreicherung der Mehrkernpolyisocyanat-Anteile.

Die Anreicherung der technisch anfallenden Polyisocyanatgemische an den erfindungsgemäß zu verwendenden Polyisocyanatgemischen gelingt nach bekannten Verfahren, z. B. durch Destillation oder über Dünnschichtverdampfer.

Destillativ kann bei einem Siedepunkt von ca. 150−200°C bei 0,1−10 Torr der Zweikerndiisocyanat-Anteil neben einem Teil des Dreikerntriisocyanat-Anteils technisch einfach abgetrennt werden. In Abhängigkeit von der Zusammensetzung des eingesetzten Polyisocyanatgemisches lassen sich die Destillationsbedingungen gewünschtenfalls so steuern, daß nur ein geringer Anteil an drei aromatische Kerne aufweisenden Triisocyanaten aus dem Gemisch entfernt wird.

Die als Rückstand anfallenden, erfindungsgemäßen Polyisocyanatgemische weisen im allgemeinen einen NCO-Gehalt von 23 bis 32, vorzugsweise 25 bis 30 Gew.-% auf. Es handelt sich um hochviskose, braune bis dunkelbraune Flüssigkeiten bzw. Harze, die in zahlreichen Lösungsmitteln, wie sie auch in lösungsmittelhaltigen Klebstoffen Verwendung finden, löslich sind. Beispiele derartiger Lösungsmittel sind Methylenchlorid, Chlorbenzol, Methylethylketon, Ethylacetat, Toluol, Xylol bzw. Gemische dieser Lösungsmittel mit sich selbst oder mit Lösungsbenzinen.

Bei der erfindungsgemäßen Verwendung der Polyisocyanatgemische gelangen diese vorzugsweise in lösungsmittelhaltigen Klebstoffen auf Basis von Natur- oder Synthesekautschuk oder von Hydroxylgruppen aufweisenden Polyurethanen zum Einsatz.

Bei den Bindemitteln für die erfindungsgemäß in Betracht kommenden Klebstoffe handelt es sich somit um

1. die üblicherweise als Klebstoff bzw. Klebstoff-Rohstoff eingesetzten Naturkautschuk-Typen;
2. die üblicherweise als Klebstoff bzw. Klebstoff-Rohstoff eingesetzten Synthesekautschuk-Typen; wie z. B. Polymerisate von Dienen wie Butadien oder Mischpolymerisate von Dienen wie Butadien mit einfach olefinisch ungesättigten Verbindungen wie z. B. Styrol, Acrylnitril oder Methacrylnitril, Polymerisate bzw. Mischpolymerisate des 2-Chlorbutadien-1,3 mit anderen olefinisch ungesättigten Monomeren beispielsweise der obengenannten Art, insbesondere eines Chlorgehalts von ca. 36 Gew.-% oder
3. die üblicherweise als Klebstoff bzw. Klebstoff-Rohstoff eingesetzten Hydroxylgruppen aufweisenden Polyurethane, des gelchromatographisch ermittelbaren Molekulargewichts von 30 000 bis 1 000 000, vorzugsweise 50 000 bis 200 000, insbesondere lineare oder weitgehend lineare, endständige Hydroxylgruppen aufweisende Polyurethane, die aus (i) Dihydroxypolyestern des rechnerischen Molekulargewichts 1250 bis 4000, vorzugsweise 2000 bis 4000, aus aliphatischen oder aromatischen Dicarbonsäuren wie Adipinsäure oder Phthalsäure und Alkandiolen wie Ethylenglykol, Tetramethylenglykol und/oder Hexamethylenglykol oder aus Polylactonen, insbesondere Poly- -caprolacton und (ii) aromatischen oder aliphatischen Diisocyanaten, insbesondere 2,4-Diisocyanatotoluol, dessen Gemischen mit 2,6-Diisocyanatotoluol, 4,4'-Diisocyanatodiphenylmethan oder Hexamethylendiisocyanat, gegebenenfalls unter Mitverwendung von (iii) Glykolen des Molekulargewichts 62−200 als Kettenverlängerungsmittel wie z. B. Tetramethylenglykol oder Hexamethylenglykol in einem molaren NCO/OH-Äquivalentverhältnis von 0,9 : 1 bis 0,999 : 1 hergestellt worden sind. Zu den erfindungsgemäß besonders bevorzugten Klebstoff-Klebemitteln gehören die beispielhaft genannten Polymerisate bzw.

Mischpolymerisate des 2-Chlorbutadien-1,3 (»Polychlorpren«) und die zuletzt genannten Hydroxylgruppen aufweisenden Polyurethane.

Die genannten Bindemittel bzw. Klebstoffrohstoffe gelangen erfindungsgemäß vorzugsweise als 10- bis 30gew.-%ige Lösungen in Lösungsmitteln bzw. Lösungsmittelgemischen der bereits beispielhaft genannten Art zum Einsatz.

Die erfindungsgemäß zu verwendenden Polyisocyanatgemische werden diesen Lösungen in Mengen von 2 bis 15 Gew.-%, bezogen auf Bindemittel der unter 1) bis 3) genannten Art zugesetzt. Um eine schnelle und homogene Durchmischung der Klebstofflösung mit dem Polyisocyanat zu erreichen, ist es oft vorteilhaft, die erfindungsgemäß zu verwendenden Polyisocyanatgemische als 10- bis 50gew.-%ige Lösung in derartigen Lösungsmitteln den Bindemittellösungen zuzusetzen.

Zur Modifizierung ihrer klebetechnologischen Eigenschaften können den Klebemitteln auch noch weitere Zusatzmittel zugemischt werden. Hierzu gehören beispielsweise Naturharze oder modifizierte Harze wie Kolophoniumester oder synthetische Harze wie Phthalatharze oder — insbesondere im Fall der Klebstoffe auf Basis von Hydroxylgruppen aufweisenden Polyurethane — auch Polymere wie z. B. Chlorkautschuk oder lösliche Polymerisate oder Mischpolymerisate des Vinylacetats oder andere Vinylverbindungen. Diese Zusatzmittel, insbesondere die beispielhaft genannten Harze werden im allgemeinen zwecks Erzielung einer besonders andauernden Kontaktbindefähigkeit oder zwecks Erhöhung der Kohäsionsfestigkeit zugesetzt.

Die die Polyisocyanatgemische enthaltenden Klebstofflösungen eignen sich zum Kleben beliebiger Werkstoffe gleicher oder verschiedener Art, z. B. zum Verkleben von Leder, Textilien, Kunststoffen, Holz und Papier, vorzugsweise zum Verkleben von Gummi- oder Weich-PVC.

Die nachstehenden Beispiele dienen zur weiteren Erläuterung der Erfindung. Alle Prozentangaben beziehen sich auf Gewichtsprozente.

## Beispiel 1

### (Herstellung eines erfindungsgemäßen Polyisocyanatgemischs)

In einem mit einer 40 cm langen Füllkörperkolonne versehenen 4-l-Dreihalskolben werden 2580 g eines durch Phosgenierung von Anilin/Formaldehyd-Kondensat erhaltenen technischen Polyisocyanatgemischs der Zusammensetzung (A) eingefüllt.

Zur Abtrennung der leichter flüchtigen Bestandteile wird bei einem Vakuum von 0,2 — 1 Torr allmählich bis auf 210° C Sumpftemperatur erhitzt.

Das Destillat geht bis zu einem Sdp. von 170° C bei 0,7 Torr über, ist schwachgelb gefärbt und kristallisiert nach kurzer Zeit.

Nachdem ca. 30% der Sumpfvorlage überdestilliert sind, wird die Destillation abgebrochen. Im Sumpf befinden sich 1720 g eines dunkelbraunen, hochviskosen Polyisocyanatgemisches, das die Zusammensetzung (B) hat.

Das Harz wird so in Methylenchlorid gelöst, daß der NCO-Gehalt der Lösung 7 Gew.-% beträgt.

| Zusammensetzung der Polyisocyanatgemische (%) | | |
|---|---|---|
| Fraktion | (A) | (B) |
| | | erfindungs-gemäß |
| Zweikern- | 26 | 3 |
| Dreikern- | 26 | 26 |
| Vierkern- | 11 | 14 |
| Höhere Anteile | 37 | 57 |

Die Zusammensetzung der Polyisocyanatgemische kann nach der Methode der Hochdruckflüssigkeitschromatographie ermittelt werden. Hierzu werden 5 µl einer 5%igen Lösung der Polyisocyanatgemische in CHCl₃ mit einem Druck von ca. 30 bar in eine µ-Styragel-Säulenkombination eingespritzt, in der die Probe nach verschiedenen Molgewichten aufgetrennt wird. 5-Kern- und höhermolekulare Fraktionen werden dabei nicht mehr getrennt und daher gemeinsam erfaßt.

# 0 028 781

Die getrennten Komponenten werden über das Brechungsinkrement durch ein Mikrorefraktometer quantitativ bestimmt.

Das mittlere Molekulargewicht des Polyisocyanatgemischs B wurde osmometrisch bestimmt. Es liegt bei 620. Der NCO-Gehalt des Gemischs liegt bei 27 Gew.-%. Hieraus errechnet sich eine mittlere NCO-Funktionalität von 3,98.

## Beispiel 2

### (Herstellung eines erfindungsgemäßen Polyisocyanatgemischs)

In das ölbeheizte Vorlagegefäß (z. B. Meßtropftrichter) eines Dünnschichtverdampfers (Länge: 22 cm, Durchmesser: 25 mm, Antrieb stufenlos regelbar bis 2000 U/min) werden 2000 g eines durch Phosgenierung von Anilin/Formaldehyd-Kondensaten erhaltenen technischen Polyisocyanatgemischs mit der Zusammensetzung (C) eingefüllt.

Mit einem Durchsatz von 200 ml/h wird das Ausgangsprodukt in den auf 220°C erhitzten Dünnschichtverdampfer, in dem ein Druck von 0,2 – 1 Torr eingestellt ist, eingetropft.

1190 g flüchtige Anteile werden über einen ölgekühlten Wärmeaustauscher kondensiert und in einem Destillationsgefäß aufgefangen.

Der Sumpf des Dünnschichtverdampfers enthält nach beendeter Fraktionierung 790 g des Polyisocyanatgemisches mit der Zusammensetzung (D). Das Polyisocyanatgemisch weist einen NCO-Gehalt von 28% auf. Das osmometrisch bestimmte mittlere Molekulargewicht des Gemischs liegt bei 640. Hieraus errechnet sich eine mittlere NCO-Funktionalität von 4,27.

Zusammensetzung der
Polyisocyanatgemische (%)

| Fraktion | (C) | (D) erfindungs- gemäß |
|---|---|---|
| Zweikern- | 49 | 3 |
| Dreikern- | 19 | 23 |
| Vierkern- | 8 | 17 |
| Höhere Anteile | 24 | 57 |

## Beispiel 3

### (Erfindungsgemäß)

Unter Verwendung der in Beispiel 1 beschriebenen Polyisocyanat-Lösung wurden 2 Klebstoffe hergestellt.

### Polyurethan-Kunststoff

Ein im wesentlichen lineares, Hydroxylgruppen-aufweisendes Polyurethan mit einem gelchromatographisch ermittelten Molekulargewicht von etwa 100 000, aus 2,4-Toluylendiisocyanat und einem Hydroxylgruppen-haltigen Polyester aus Adipinsäure und Ethylenglykol wurde in Methylethylketon zu einer etwa 20%igen Lösung mit einer Viskosität von 2 Pa · s bei 20°C gelöst. 100 Teile dieser Polyurethan-Lösung wurden mit 10 Teilen der Polyisocyanat-Lösung gründlich gemischt.

### Polychloropren-Klebstoff

50 Teile Chloropren-Kautschuk starker Kristallisationsneigung (Chlorgehalt: 36%) mit einem Mooney-Wert von 84 nach DIN 53 523 und 50 Teile Chloropren-Kautschuk mittlerer Kristallisationsneigung (Chlorgehalt: 36%) mit einem Mooney-Wert von 100 wurden gemeinsam auf einem mit Wasser gekühlten Walzwerk gründlich mastiziert. Während der Mastikation wurden je 4 Teile Magnesiumoxid

5

und Zinkoxid eingearbeitet. Das entstandene Walzfell wurde in einem Gemisch aus Ethylacetat, Benzin (Siedebereich 65 (95°C)) und Toluol im Gewichtsverhältnis 2 : 2 : 1 zu einer Viskosität von 2 Pa · s bei 20°C gelöst. Der Feststoffgehalt der Lösung betrug etwa 23%.

100 Teile dieser Lösung wurden mit 10 Teilen der Polyisocyanatlösung gemischt.

Die Prüfung der beiden Klebstoffe erfolgte auf den im folgenden beschriebenen 4 Testmaterialien, die gebräuchlichen Sohlenmaterialien entsprechen.

Test-Material A:   Naturkautschuk-Gummisohlenmaterial mit einem Gehalt an Silikat-Füllstoff von 40%.
Shore-Härte A 70 nach DIN 53 505.

Test-Material B:   Styrol-Butadien-Gummisohlenmaterial mit einem Gehalt an Silikat-Füllstoff von etwa 40%.
Shore-Härte A 70 nach DIN 53 505.

Test-Material C:   Ölgestreckter Styrol-Butadien-Kautschuk mit einem Ölgehalt von 37,5% und einem Gehalt an Silikat-Füllstoff von 40%.
Shore-Härte A 60 nach DIN 53 505.

Test-Material D:   Naturkautschuk-Sohlenmischung mit einem Anteil von 20% eines hochstyrolhaltigen Harzes. Gehalt an Silikat-Füllstoff 60%.
Shore-Härte A 90 nach DIN 53 505.

Zur Prüfung der Klebstoffe wurden aus den Testmaterialien nach DIN 53 273 Prüfkörper hergestellt. Vor dem Aufbringen des Klebstoffes wurde das Gummimaterial mit Schleifpapier der Körnung 40 gründlich gerauht. Der Klebstoffauftrag erfolgte beidseitig zweimal, so daß auf jeder Seite ca. 50 g/m², bezogen auf Feststoff, des Klebstoffs vorlagen. Anschließend wurden die Klebschichten 4 Minuten mit 250-Watt-Infrarotlampen in einer Entfernung von 25 cm bestrahlt, dann die Klebung zusammengelegt und 5 Minuten mit 0,35 MPa gepreßt. Nach dem Kleben wurden die Prüfkörper zunächst 9 Tage bei 20°C gelagert. Der im Trennversuch bei einem Spindelvorschub von 100 mm/min nach DIN 53 273 bei 23°C ermittelte Schälwiderstand ist in nachfolgender Tabelle aufgeführt:

Schälwiderstand N/mm

|  | Testmaterial | | | |
|  | A | B | C | D |
| --- | --- | --- | --- | --- |
| PU-Klebstoff | 5,4 | 6,8 | 5,6 | 9,0 |
| CR-Klebstoff | 4,1 | 5,0 | 3,6 | 4,2 |

Zur Ermittlung der Wärmefestigkeit wurden 9 Tage gelagerte Prüfkörper einem Zeitstandschälversuch unterworfen. Hierbei wurde die Zeit ermittelt, während der die Klebung der angegebenen Last von 10 N/cm und bei einer Temperatur von 50°C widerstanden. Die angefallenen Ergebnisse sind in nachfolgender Tabelle aufgeführt:

Zeitstandschälversuch bei 50°C (min)

|  | Testmaterial | | | |
|  | A | B | C | D |
| --- | --- | --- | --- | --- |
| PU-Klebstoff | 68 | 85 | 58 | 180 |
| CR-Klebstoff | 18 | 11 | 9 | 102 |

Die Wärmefestigkeit von frischen Klebungen wurde unmittelbar nach ihrer Herstellung im Schälversuch ermittelt. Hierzu wurden Nora-Gummi-Klebungen sofort nach ihrer Herstellung 1 h bei 50°C gelagert. Der Schälwiderstand wurde sofort an dem noch 50°C warmen Prüfkörper ermittelt.

6

Schälwiderstand bei 50°C [N/mm]

| | Testmaterial |
| --- | --- |
| | D |
| PU-Klebstoff | 2,5 |

Die Klebungen verfärben sich auch nach längerer Lagerzeit nicht.

### Vergleichsversuch 1

In diesem und dem nachstehenden Vergleichsversuch 2 wurden die gleichen Mengen an Klebstoff bzw. Polyisocyanat wie in Beispiel 3 verwendet.

Als Polyisocyanat wurde in diesem Vergleichsversuch 1 das in Beispiel 1 (Zusammensetzung A) beschriebene Produkt verwendet. Die Prüfung erfolgte nach den in Beispiel 3 angegebenen Methoden.

Schälwiderstand N/mm

| | Testmaterial | | | |
| --- | --- | --- | --- | --- |
| | A | B | C | D |
| PU-Klebstoff | 4,8 | 4,2 | 2,8 | 6,3 |
| CR-Klebstoff | 3,8 | 4,0 | 3,0 | 4,0 |

Zeitstandschälversuch bei 50°C (min)

| | Testmaterial | | | |
| --- | --- | --- | --- | --- |
| | A | B | C | D |
| PU-Klebstoff | 8 | 6 | 9 | >180 |
| CR-Klebstoff | 4 | 5 | 4 | 44 |

Schälwiderstand bei 50°C [N/mm]

| | Testmaterial |
| --- | --- |
| | D |
| PU-Klebstoff | 1,1 |

### Vergleichsversuch 2

Als Polyisocyanat wurde eine 20gew.-%ige Lösung von Tris-(p-isocyanatophenyl)-methan in Methylenchlorid verwendet. Der NCO-Gehalt betrug 7 Gew.-%. Die Prüfung erfolgte nach den im Beispiel 3 angegebenen Methoden.

7

Schälwiderstand N/mm

| Testmaterial | | | |
|---|---|---|---|
| A | B | C | D |
| PU-Klebstoff 5,1 | 6,5 | 5,4 | 7,6 |
| CR-Klebstoff 4,0 | 5,2 | 4,0 | 4,2 |

Zeitstandschälversuch bei 50°C (min)

| Testmaterial | | | |
|---|---|---|---|
| A | B | C | D |
| PU-Klebstoff 73 | 96 | 65 | >180 |
| CR-Klebstoff 18 | 12 | 7 | 117 |

Schälwiderstand bei 50°C N/mm

| Testmaterial |
|---|
| D |
| PU-Klebstoff 2,5 |

Die Klebungen verfärben sich nach mehrtägiger Lagerung dunkelviolett.

**Patentansprüche**

1. Polyisocyanatgemische der Diphenylmethanreihe, gekennzeichnet durch

a) eine mittlere NCO-Funktionalität von 3,5 bis 5 und
b) einen Gehalt an
   Zweikernisocyanaten von 0 bis 15 Gew.-%,
   Dreikernisocyanaten von 10 bis 45 Gew.-%,
   Vierkernisocyanaten von 5 bis 30 Gew.-% und
   höherkernigen Polyisocyanaten von 30 bis 80 Gew.-%, wobei sich die genannten Prozentsätze zu
   100 Gew.-% ergänzen.

2. Verwendung von Polyisocyanatgemischen der Diphenylmethanreihe mit

a) einer mittleren NCO-Funktionalität von 3,5 bis 5 und
b) einem Gehalt an
   Zweikernisocyanaten von 0 bis 15 Gew.-%,
   Dreikernisocyanaten von 10 bis 45 Gew.-%,
   Vierkernisocyanaten von 5 bis 30 Gew.-% und
   höherkernigen Polyisocyanaten von 30 bis 80 Gew.-%, wobei sich die genannten Prozentsätze zu
   100 Gew.-% ergänzen,
als Zusatzmittel in Klebstoffen auf Basis von Natur- oder Synthesekautschuk oder Hydroxylgruppen
aufweisenden Polyurethanen.

**0 028 781**

**Claims**

1. Polyisocyanate mixtures of the diphenyl methane series, characterised by

a) an average NCO-functionality of 3.5 to 5 and
b) a content of
binuclear isocyanates of 0 to 15% by weight,
trinuclear isocyanates of 10 to 45% by weight,
tetranuclear isocyanates of 5 to 30% by weight and
higher polyisocyanates of 30 to 80% by weight, the stated percentages totalling 100% by weight.

2. Use of polyisocyanate mixtures of the diphenyl methane series having

a) an average NCO-functionality of 3.5 to 5 and
b) a content of
binuclear isocyanates of 0 to 15% by weight,
trinuclear isocyanates of 10 to 45% by weight,
tetranuclear isocyanates of 5 to 30% by weight and
higher polyisocyanates of 30 to 80% by weight, the stated percentages totalling 100% by weight,
as additives in adhesives based on natural or synthetic rubber or hydroxyl-group-containing polyurethanes.


**Revendications**

1. Mélanges de polyisocyanates de la série du diphénylméthane, caractérisés par

a) une fonctionnalité NCO moyenne de 3,5 à 5 et
b) une teneur en
isocyanates à deux noyaux de 0 à 15% en poids,
une teneur en isocyanates à trois noyaux de 10 à 45% en poids,
une teneur en isocyanates à quatre noyaux de 5 à 30% en poids et
une teneur en polyisocyanates à plus grand nombre de noyaux de 30 à 80% en poids, les pourcentages mentionnés se complétant à 100% en poids.

2. Utilisation de mélanges de polyisocyanates de la série du diphénylméthane ayant

a) une fonctionnalité NCO moyenne de 3,5 à 5 et
b) une teneur en isocyanates à deux noyaux de 0 à 15% en poids,
une teneur en isocyanates à trois noyaux de 10 à 45% en poids,
une teneur en isocyanates à quatre noyaux de 5 à 30% en poids et
une teneur en polyisocyanates à plus grand nombre de noyaux de 30 à 80% en poids,
les pourcentages mentionnés se complétant à 100% en poids, comme additifs dans des adhésifs à base de caoutchouc naturel ou synthétique ou de polyuréthannes porteurs de groupes hydroxyle.

9